(19) Europäisches Patentamt / European Patent Office / Office européen des brevets

(11) **EP 4 141 878 A1**

(12) **EUROPEAN PATENT APPLICATION**

(43) Date of publication:
**01.03.2023 Bulletin 2023/09**

(21) Application number: **21193589.5**

(22) Date of filing: **27.08.2021**

(51) International Patent Classification (IPC):
*G16H 15/00* (2018.01)

(52) Cooperative Patent Classification (CPC):
**G16H 30/40; G16H 15/00; G16H 50/20;
G16H 50/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Siemens Healthcare GmbH
91052 Erlangen (DE)**

(72) Inventor: **Conjeti, Sailesh
91056 Erlangen (DE)**

Remarks:
Amended claims in accordance with Rule 137(2)
EPC.

(54) **CUE-BASED MEDICAL REPORTING ASSISTANCE**

(57) Various examples relate to a workflow for drawing up a medical report (145) of a medical examination. One or more diagnostic cues (119) are posed to the user, to thereby assist drawing up the medical report.

## FIG 1

EP 4 141 878 A1

**Description**

Various examples of the disclosure pertain to a workflow for drawing up a medical report. Various examples of the disclosure specifically relate to one or more diagnostic cues being posed to the user.

Medical practitioners are trained in drawing up medical reports for patients based on a diagnosis. Typically, the diagnosis is based on medical imaging datasets, obtained by medical measurements such as tomography.

There is a need for advanced techniques of assisting medical practitioners in drawing up medical reports.

This need is met by the features of the independent claims. The features of the dependent claims define embodiments.

Techniques are disclosed that pose one or more diagnostic cues to a user as part of a workflow for drawing up a medical report. The one or more diagnostic cues can assist the user, e.g., a medical practitioner, in drawing up the medical report.

A computer-implemented method is provided. The method includes obtaining medical imaging dataset of a current examination of a patient. The method further includes, based on the medical imaging dataset, determining one or more diagnostic cues using a processing algorithm, the one or more diagnostic cues being associated with patient-specific diagnostic findings. The method further includes, as part of a workflow for drawing up a current medical report for the current examination, controlling a user interface to pose the one or more diagnostic cues to a user.

A computer program or a computer program product or a computer-readable storage medium includes program code. The program code can be loaded an executed by at least one processor. Upon executing the program code, the at least one processor performs a method. The method includes obtaining medical imaging dataset of a current examination of a patient. The method further includes, based on the medical imaging dataset, determining one or more diagnostic cues using a processing algorithm, the one or more diagnostic cues being associated with patient-specific diagnostic findings. The method further includes, as part of a workflow for drawing up a current medical report for the current examination, controlling a user interface to pose the one or more diagnostic cues to a user.

A device includes a processer. The processor is configured to obtain medical imaging dataset of a current examination of a patient. The processor is configured to determine one or more diagnostic cues based on the medical imaging dataset and using a processing algorithm, the one or more diagnostic cues being associated with patient-specific diagnostic findings. The processor is also configured to, as part of a workflow for drawing up a current medical report for the current examination, control a user interface to pose the one or more diagnostic cues to a user.

It is to be understood that the features mentioned above and those yet to be explained below may be used not only in the respective combinations indicated, but also in other combinations or in isolation without departing from the scope of the invention.

FIG. 1 schematically illustrates a data processing for drawing up a medical report according to various examples.

FIG. 2 schematically illustrates a device according to various examples.

FIG. 3 is a flowchart according to various examples.

FIG. 4 schematically illustrates a workflow according to various examples.

FIG. 5 is a flowchart of a method according to various examples.

FIG. 6 schematically illustrates a processing algorithm for determining one or more diagnostic cues according to various examples.

FIG. 7 schematically illustrates a processing algorithm for determining one or more diagnostic cues according to various examples.

FIG. 8 schematically illustrates a processing algorithm for determining one or more diagnostic cues according to various examples.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques of assisting medical practitioners in drawing up medical reports are presented. Specifically, the techniques disclosed herein include posing one or more diagnostic cues to a user. The one or more diagnostic cues are based on a medical imaging dataset of a current examination of the patient. The one or more diagnostic cues may be optionally based on at least one prior medical report of at least one prior medical examination.

The one or more diagnostic cues can be associated with patient-specific diagnostic findings.

For example, the diagnostic cues could be formulated/posed as questions, reports, snippets, etc. The diagnostic cues can be comparably short sentences that convey information content.

As a general rule, the one or more diagnostic cues could point towards - i.e., include a semantic content associated with - or query characteristic features associated with a pathology or abnormalities included in a medical imaging dataset. For instance, diagnostic cues could point towards or query diagnostic root causes of such abnormality. For instance, diagnostic cues could point towards or query follow-up examinations and/or treatments.

The diagnostic cues can be patient specific. I.e., the diagnostic cues can be determined to relate to a patient-specific characteristics. Different examinations with different patients may be associated with different diagnostic cues.

Posing one or more such diagnostic cues can help to engage in an interaction with the user, so as to ensure that the medical report is comprehensive and error-free.

Specifically, the medical report can be generated and/or revised (edited) taking into account the one or more diagnostic cues. This may be based on an automated algorithmic implementation, or based on an appropriate configuration of a user interface (UI) that enables the medical practitioner to take into account the one or more diagnostic cues.

As a general rule: Medical reports could be radiology reports or other text-based electronic medical data such as lab-diagnostics results, clinical notes, surgical records, discharge records, and pathology reports.

To pose the one or diagnostic cues, the UI can be controlled. A graphical UI can be controlled. In particular, the one or more diagnostic cues can be posed as part of the workflow for drawing up the medical report. The workflow for drawing up the medical report can include an interaction framework for the medical practitioner to include relevant information in the medical report. For instance, it would be possible that the one or more cues are linked to certain sections of the medical report that has been pre-generated (if the one or more diagnostic cues are posed to the user prior to a generation step of the workflow). The medical report may be shown face-to-face with the one or more diagnostic cues. The one or more diagnostic cues may be graphically associated with the sections of the medical report.

Thereby, an interactive process can be triggered that enables the medical practitioner to comprehensively and accurately draw up the medical report based on the semantic guidance provided by the one or more diagnostic cues.

As a general rule, there are various options available for implementing said posing of the one or more diagnostic cues. Some options are summarized in TAB. 1 below.

TAB. 1: Various options for implementing diagnostic cues. The options can also be combined with each other.

| | Example implementation | Example details |
|---|---|---|
| I | Question | The diagnostic cues can be posed as questions. This means that the user can have the possibility to answer a medical question via the UI. The method may accordingly include obtaining, from the UI, a user feedback to at least one of the one or more medical cues. Thus, additional information relevant for drawing up the medical report may be collected. This user feedback may be used to edit the medical report. |

(continued)

|  | Example implementation | Example details |
|---|---|---|
|  |  | For instance, "yes/no" questions may be posed. It would be possible to acquire quantitative feedback, e.g., pertaining to the size of certain anatomical features. |
|  |  | It would be possible that user feedback is obtained via the UI in response to posing questions. This user feedback may then be used as part of the workflow for drawing up the medical report. The medical report may be edited based on the user feedback. For instance, it would be possible that the medical report is generated based on the user feedback. Here, the one or more questions can be posed to the user prior to a generating step of the workflow for drawing up the medical report. Also, in scenarios where the medical report has been pre-generated, it would be possible that the medical report is revised based on the user feedback. |
| II | Notes | It would be possible that the diagnostic cues are posed as notes. Here, it is not required to obtain explicit user feedback. Rather, the user may implicitly consider the notes and use them, as deemed appropriate. |
|  |  | For instance, the notes can be used as checklist or "safety net". |
|  |  | The diagnostic cues can, accordingly, provide context information that can be helpful to the medical practitioner when drawing up the medical report. For instance, it would be possible that the one more diagnostic cues are posed as notes after a generation step over the workflow for drawing up the medical report. Thereby, a validation of the medical report - e.g., for completeness and/or correctness - may be facilitated by the one or more diagnostic cues. |
|  |  | The one or more diagnostic cues could be determined based on the medical report that has been pre-generated. I.e., it would be possible that the one or more diagnostic user tailored to the medical report. Thereby, for instance, when the user manually draws up the medical report, during the validation step, the user can be confronted with the one or more diagnostic cues that act as a checklist for completeness or serve to cross checked the correctness of content of the medical report. |
|  |  | Based on the one or diagnostic cues, the medical practitioner may check whether all information is contained in the medical report, or with additional information is required. The medical practitioner may check whether the one or diagnostic cues are consistent or inconsistent with the medical report. Thereby, the validation step of the workflow may provide for a validation toolset for the medical practitioner to validate the medical report. |

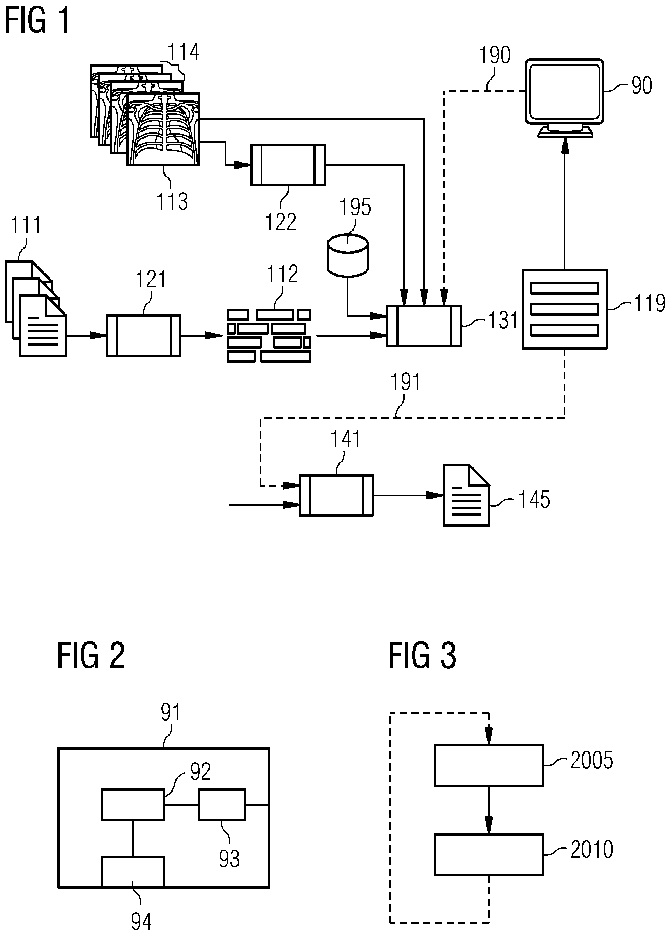

FIG. 1 schematically illustrates data processing according to various examples. FIG. 1 illustrates aspects with respect to one or more cues 119 that are posed to a user as part of a workflow for drawing up a current medical report 119. The one or more cues 119 are determined using a processing algorithm 131.

As illustrated in FIG. 1, at least one prior medical report 111 of at least one prior examination of the patient is obtained (this is, however, generally optional). For instance, the at least one medical report 111 could be retrieved from a medical repository of the hospital. It could be retrieved from a server.

Using the at least one prior medical report 111 of the at least one prior examination in connection with drawing up the current medical report facilitates a longitudinal analysis. For instance, the development of a pathology of the patient may be assessed. In particular, the one or more diagnostic cues can pertain to such development of a pathology between the at least one prior examination and the current examination.

Then, using a natural language processing algorithm 121, the at least one medical report 111 may analyzed to obtain a machine-readable representation 112 of the at least one prior medical report. This serves as an input to the processing algorithm 131. As a general rule, using the natural language processing algorithm 121 is optional. It would be possible that the processing algorithm 131 directly operates based on the at least one prior medical report 111.

The processing algorithm 131 also obtains, as an input, a current medical imaging dataset 113 or result data that is determined by a medical analytics algorithm 122 based on the current medical imaging dataset 113. The processing algorithm 131 thus also operates based on a medical imaging dataset 113 of the current examination of the patient.

Alternatively or additionally to the current medical imaging dataset 113, it would also be possible to obtain at least one medical imaging dataset 114 of the at least one prior examination (e.g., the same at least one prior examination to which the at least one prior medical report 111 pertains to, or another prior examination). The one or more diagnostic cues 119 can be determined further based on the at least one prior medical imaging dataset.

Illustrated in FIG. 1 are two options for taking into account the current medical imaging dataset 113 and/or the at least one prior medical imaging dataset 114. In one option, it is possible that the processing algorithm 131 that determines the one or more diagnostic cues 119 directly operates based on the respective imaging dataset or datasets 113, 114. Alternatively or additionally, it would also be possible to analyze the current medical imaging dataset 113 and/or the at least one prior medical imaging dataset 114 using a medical analytics algorithm 122 to determine result data for the respective medical imaging dataset. Then, the one or more diagnostic cues 119 can be determined based on the result data.

The current and/or prior medical imaging dataset could be one or more of the following: magnetic resonance tomography images; computed tomography images; OCT images; images obtained by photoacoustic tomography; to determine emission tomography images; SPECT images; images obtained from diffuse scattering tomography; infrared tomography images; ultrawide bandwidth tomography images; ultrasound images; echocardiogram data; images of histopathology samples; stained tissue samples; etc. The techniques can apply to radiology or other disciples such as oncology, dermatology, ophthalmology etc. and is not limited to radiological images.

The processing algorithm 131 can, optionally, take into account one or more parameters. Such parameters can be obtained from a database 195. One example from each of the be an institution-specific reporting guidelines for drawing up medical reports. In other words, it would be possible that the processing algorithm 131 determines the one or diagnostic cues 119 in accordance with the institution-specific reporting guidelines for drawing up medical reports. Respective parameterization data can be obtained from the database 195 and fed to a respective input channel of the processing algorithm 131. It would also be possible that the processing algorithm 131 is fixedly trained to determine the one or more diagnostic cues 119 in accordance with a single institution-specific reporting guideline. Thereby, the processing algorithm 119 can also be built on Clinical Reporting Guidelines for individual modalities such as Bi-RADS, Pi-RADS, LungRADS to name a few.

Once the processing algorithm 131 has determined the one or more diagnostic cues 119, it is then possible to control a UI 90 as part of a workflow for drawing up the current medical report 145. The UI 90 is controlled to pose the one or more diagnostic cues (cf. TAB. 1).

For instance, the posing can be triggered in the backend as soon as a particular case is sent to the radiology worklist. Once the case is opened for reporting, the one or more diagnostic cues 119 can either be shown on the PACS / RIS window, or as a pop-up message or before case-closeout as a confirmatory step. Alternatively, posing the one or more diagnostic cues can be triggered on-demand in response to one or more trigger criteria. For instance, posing the one or more cues can be triggered if it is judged that diagnostic errors / blanks exist in the medical report 145; then an associated diagnostic cue can be posed to the user.

Where multiple diagnostic cues 119 can be posed to the user, it would be possible that the multiple diagnostic cues 119 art posed all in parallel to the user. It would also be possible that the multiple cues are sequentially posed to the user.

In some examples, it would be possible that the multiple cues 119 are sequentially determined. Specifically, it would be possible to obtain a user feedback associated with a first one of the multiple cues. Then, a second one of the multiple cues can be determined, using the processing algorithm 131 considering the user feedback (cf. FIG. 1, user feedback 190) .

Depending on the implementation, it would then be possible that the user interactively considers these one or more diagnostic cues 190 when drawing up the medical report 145. It would also be possible that user feedback 191 is obtained, e.g., where the one or more diagnostic cues are implemented as questions. Then, using an appropriate algorithm 141, the medical report 145 may be generated or, at least, revised based on the user feedback 191. Here, further input may be considered, e.g., the medical imaging dataset 113 and/or at least one prior medical report 111.

Summarizing, by means of the data processing of FIG. 1, a radiology question generation engine can be implemented which takes in unstructured free text of past radiology reports 111, prior images 114 and a current image 113 and provides a list of actionable questions 119 to be posed to the radiologist while reporting on the current image. Thereby, a more accurate and comprehensive diagnosis can be facilitated. The one or more diagnostic cues 119 also support medical education by acting as a virtual pedagogical teacher posing pertinent questions to junior radiologists / residents. It can aid in clinical decision making and in patient education to help patients pose the right clinical questions to their clinicians.

FIG. 2 schematically illustrates a device 91 that can implement the processing according to FIG. 1, at least in parts. The device 91 includes a processor 92 in the memory 93, as well as an interface 94. For instance, the processor 92 can receive medical imaging datasets 113, 114 and/or at least one prior medical report 111 via the interface 94. The processor 92 can load and execute program code from the memory 93. Loading and executing the program code causes the processor 92 to perform techniques as disclosed herein, e.g., techniques associated with executing a workflow for drawing up medical report, controlling a UI 90, posing one more diagnostic cues 119 that have been determined based on executing a processing algorithm 131, image preprocessing using a medical analytics algorithm 122, editing a medical report, training a processing algorithm for determining one or more diagnostic cues, etc.

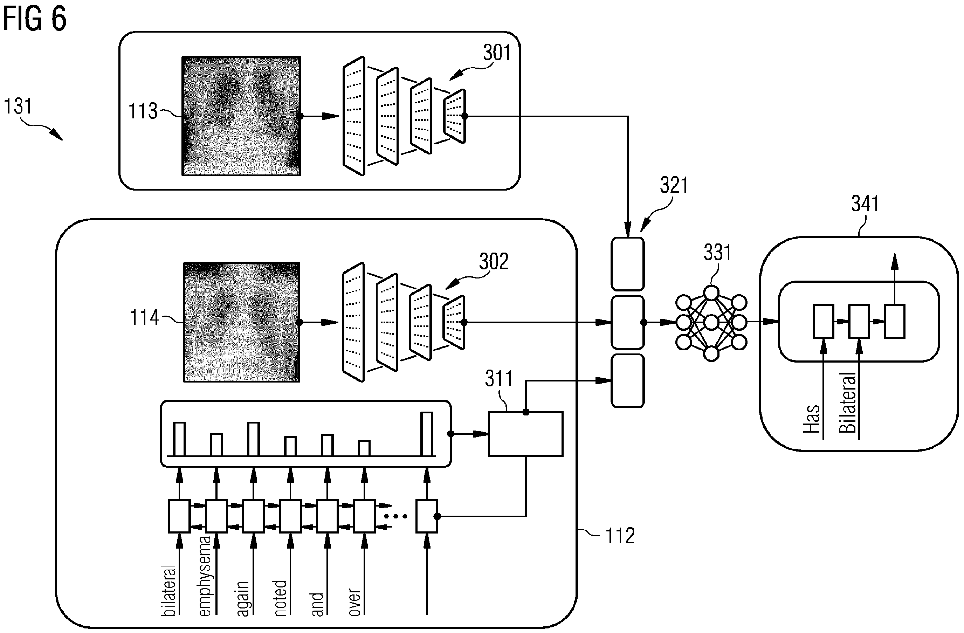

As a general rule, various options are available for implementing the processing algorithm 131. Specifically, it would be possible that the processing algorithm 131 is implemented as a machine-learning algorithm. Here, in a training phase 2005 (cf. FIG. 3; further details will be explained in connection with FIG. 6 below for a specific example implementation of the processing algorithm 131), the machine-learning processing algorithm 131 can be trained. For this, appropriate training data can be considered, the training data including a training input and a ground-truth label. Then, using techniques such as gradient descent optimization and/or back propagation, it is possible to adjust weights of the machine-learning processing algorithm 131, during the training phase 2005.

Once the machine-learning processing algorithm 131 has been appropriately trained, it is possible to implement an inference phase 2010. Here, the one or diagnostic cues 119 can be predicted based on input data as discussed above in connection with FIG. 1.

In some examples, the training phase 2005 may be re-executed from time to time, based on ground-truth labels obtained from or during the inference phase 2010. This is a technique that can be labeled life-long-learning (LLL). It is illustrated in FIG. 3 using the dashed feedback arrow.

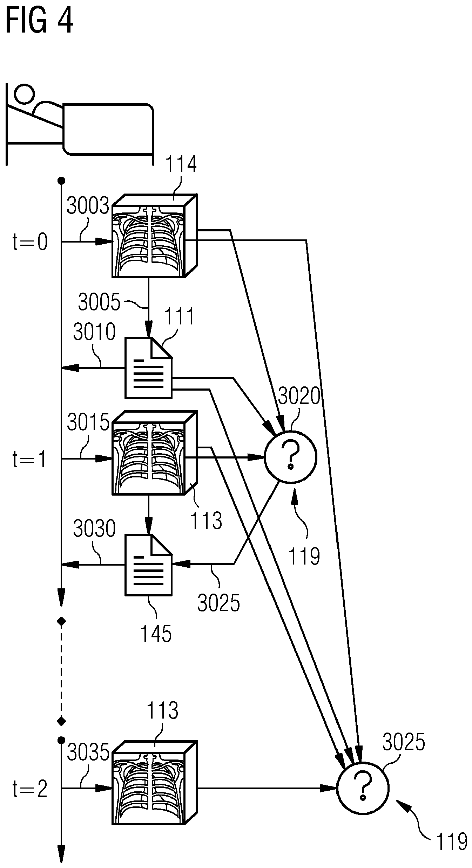

FIG. 4 schematically illustrates a diagnostic workflow according to various examples. Various actions are resolved over time (from top to bottom in FIG. 4).

At 3003, a medical imaging dataset 114 is acquired (at time t=0).

According to the examples disclosed herein, a medical imaging dataset could include one or more of the following: an x-ray image; an ultrasound image; a blood analysis; a urine analysis; a computed tomography image; a magnetic resonance tomography image; to give just a few examples.

At 3005, a medical report 111 is drawn up based on the medical imaging dataset 114, e.g., manually by a medical practitioner or aided by an algorithm (cf. FIG. 1, algorithm 141). For instance, the medical report 111 can be drawn up by a first radiologist.

The medical report 111 is stored at 3010.

At a later point in time (t=1), at 3015, the current medical imaging dataset 113 is acquired.

Based on this, at 3020, one on more diagnostic cues that are associated with patient-specific diagnostic findings determined based on the current medical imaging dataset 113 are posed to the user, e.g., a second radiologist. This occurs during a workflow for drawing up, at 3025, a current medical report 145.

The current medical report 145 is then stored at 3030.

The process can be again repeated at 3025 using a further current medical imaging dataset 113.

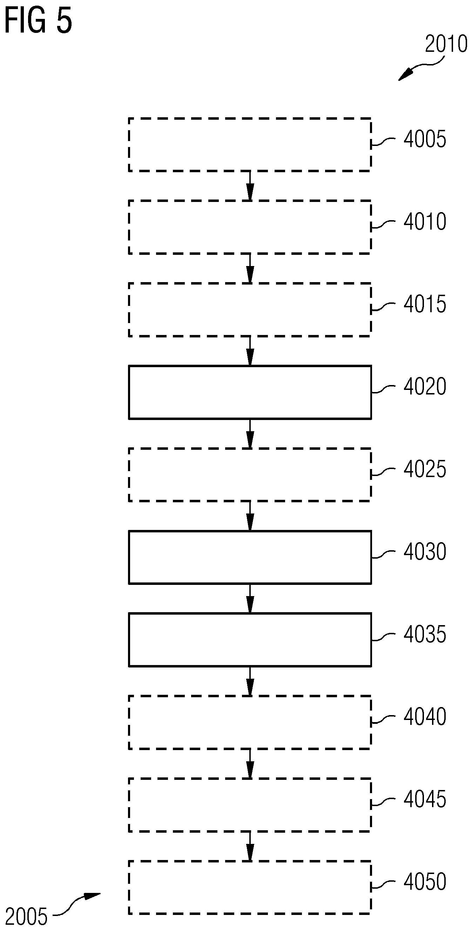

FIG. 5 is a flowchart of a method according to various examples. The method of FIG. 5 facilitates support of a medical practitioner when drawing up a medical report. The method of FIG. 5 can implement, e.g., the data processing according to FIG. 1. The method of FIG. 5 can implement parts of the workflow of FIG. 4, e.g., those parts for drawing up the medical report 145 at 3015, 3020, 3025, 3030.

Optional boxes are labeled with dashed lines in FIG. 5.

For instance, the method of FIG. 5 could be executed by a processing device such as the device 91. For instance, the processing of FIG. 5 could be implemented by the processor 92 upon loading program code from the memory 93 and upon executing the program code.

At optional box 4005, at least one prior medical report of at least one prior examination of a patient is obtained. It would be possible to obtain a series of prior medical reports of the patient, e.g., as discussed in connection with FIG. 4. A longitudinal analysis can thereby be facilitated.

It would be possible to obtain the at least one prior medical report from an Electronic Health Records, from a hospital information system, from a radiology information system, to give just a few examples.

Medical reports can be written free text and are typically organized into multiple sections such as background, findings, and impression.

The at least one prior medical report may be loaded via an interface, e.g., the interface 94 of the device 91 (cf. FIG. 2).

At optional box 4010, it is then possible to use a natural language processing algorithm to analyze the at least one prior medical report to obtain a machine-readable representation of the at least one prior medical report (cf. FIG. 1): machine-readable representation 112.

For instance, it would be possible that the machine-readable representation classifies multiple sections of the at least one prior medical report. This, later on, facilitates providing different ones of the multiple sections to different input channels of the processing algorithm that is used to determine the one or more diagnostic cues.

As a general rule, various options are available for preprocessing, using the natural language processing algorithm, the at least one prior medical report. For instance, the at least one prior medical report may be tokenized and parsed into sections corresponding to findings, backgrounds, indications, and summary.

This can be achieved using techniques such as named entity recognition, parts-of-speech tagging, lower-casing and removing stop words. Named entities can be optionally replaced with respective tags (such as RadLex mapping / CT-SNOMED mapping) to allows for better model generalization and learn patterns in the data.

An example is shown in TAB. 2.

TAB. 2: Example parsing and tokenization of free text in a prior medical report.

| "Bilateral emphysematous again noted and lower lobe fibrotic changes. Postsurgical changes of the chest including cabg procedure, stable." | "[[RID5771]] [[RID4799]] again noted and [[RID34696]] [[RID3820]] changes. Postsurgical changes of the [[RID1243]] including [[RID35862]] procedure, stable." |
| --- | --- |

To enable knowledge transfer from a large corpus of related medical data, at box 4010 it is possible to use word vectors from methods such as GloVE (Pennington, J., Socher, R. and Manning, C.D., 2014, October. Glove: Global vectors for word representation. In Proceedings of the 2014 conference on empirical methods in natural language processing (EMNLP) (pp. 1532-1543)), or language-models such as Bio-BERT (Lee, J., Yoon, W., Kim, S., Kim, D., Kim, S., So, C.H. and Kang, J., 2020. BioBERT: a pre-trained biomedical language representation model for biomedical text mining. Bioinformatics, 36(4), pp.1234-1240.) to name a few.

At optional box 4015, it is possible to obtain at least one prior medical imaging dataset (cf. FIG. 1: prior medical imaging datasets 114).

It would be possible to load such at least one prior medical imaging dataset from a picture archiving system or, generally, from a patient data repository.

At box 4020, it is then possible to obtain a current medical imaging dataset that is associated with a current examination of the patient. The current medical imaging dataset serves as the basis for the medical report to be drawn up.

It would be possible to load such at least one current medical imaging dataset from a picture archiving system or, generally, from a patient data repository. A medical imaging device may be controlled to acquire the current medical imaging dataset.

At optional box 4025, it would be possible to preprocess/ analyze all or at least one of the available medical imaging datasets, using an appropriate medical analytics algorithm. Respective techniques have been discussed in connection with FIG. 1: medical analytics algorithm 122.

Various medical analytics algorithms are known. Such medical analytics algorithms can extract one more quantitative or qualitative properties from medical imaging datasets. For instance, medical analytics algorithms are known that detect liaisons or tumors. Medical analytics algorithms are known that detect abnormal anatomic conditions. The particular implementation of the medical analytics algorithm is not germane for the functioning of the techniques disclosed herein; as such, prior art implementations of medical analytics algorithms can be used.

Then, at box 4030, it is possible to determine one or more diagnostic cues that are associated with patient-specific diagnostics findings. This is based, at least, on the medical imaging dataset that is obtained at box 4020, and potentially the output of the medical analytics algorithm executed at box 4025. Optionally, the determining of the one or more diagnostic cues could also be based on the at least one prior medical report as obtained in box 4005 and as optionally preprocessed in box 4010.

In some examples, the current medical report can be pre-generated, e.g., manually by the user. In such examples, it would be possible that the one or more diagnostic cues are determined based on the pre-generated current medical report. Note that even though the current medical report has been pre-generated, it is possible to subsequently revise the pre-generated current medical report, based on the one or more diagnostic cues.

At box 4035, the one or more diagnostic cues are posed to the user. A UI can be controlled accordingly.

Optionally, at box 4040 user feedback regarding the semantic content of the one or more diagnostic cues can be obtained, e.g., the one or more cues can be posed as questions (cf. FIG. 1: feedback 190, 191).

It would be optionally possible that the medical report is generated and/or revised (edited) based on user feedback of box 4040. In other examples, it would also be possible that the user manually draws of the medical report.

Optionally, at box 4050, LLL could be implemented, as part of the training phase 2005. This can be based on further user feedback regarding a quality of the one or more cues that are posed to the user at box 4035. A "like" or "dislike" button may be used. A "1 to 5 star rating" or the like may be used. Thereby, ground-truth labels for the training can be derived. Various options are available for implementing the processing algorithm used at box 4030 to determine the one or more diagnostic cues. An example implementation as illustrated in FIG. 6.

FIG. 6 schematically illustrates an example implementation of the processing algorithm 131 used to determine one or more diagnostic cues. In the example of FIG. 6, the processing algorithm 131 is a machine-learning algorithm. This means that weights / parameter values of the machine-learning algorithm can be set in a training process 2005 using optimization techniques based on a ground-truth label.

The machine-learning algorithm 131 is based on a neural-network architecture. The processing algorithm 131 includes multiple encoder branches 301, 302, 311. Each encoder branch determines respective latent features 321.

The encoder branches can each include multiple subsequent layers for contracting the dimensions of the input data (encoding).

For instance, the encoder branch 301 determines latent features 321 for the current medical imaging dataset 113.

For instance, the encoder branch 302 determines latent features 321 for the previous medical imaging dataset 114.

For instance, the encoder branch 311 determines latent features 321 for the at least one prior medical report. In the illustrated example, the encoder branch 300 operates based on a machine-readable representation 112.

For instance, a recurrent neural network (RNN) such as a bidirectional long-short-term memory encoder branch 311 may be used for determining the latent features 321 of the machine-readable representation 112.

Within the RNN-encoder, an attention layer can be used (not illustrated in FIG. 6). The attention layer can determine shortcuts between respective latent features 321 and a respective input to that encoder branch. Such attention mechanism may be implemented in accordance with : Yu, Z., Yu, J., Cui, Y., Tao, D. and Tian, Q., 2019. Deep modular co-attention networks for visual question answering. In Proceedings of the IEEE/CVF Conference on Computer Vision and Pattern Recognition (pp. 6281-6290).

Thereby, the diagnostic cue generation can focus on appropriate named entities and relationships; rather than on semantic variations due to individual reporting styles.

The encoder branches 301, 302 can be implemented as a convolutional neural network (CNN). Here, in convolutional layers, input weights of convolved with a predefined kernel for each layer. The weights of the kernel are trained in the training phase 2005.

The CNN encoder can be represented using deep CNNs such as deep belief network, ResNet, DenseNet, Autoencoders, capsule networks, generative adversarial networks, Siamese networks, convolutional neural networks, variational autoencoders, image transformer networks etc.

To ensure that the generated cues are intelligible, templates can be used. Some question templates are shown in TAB. 3 below.

TAB. 3: example cues templates, here for an implementation of posing the cues as questions as in TAB. 1 example I. #### is the placeholder for tokenized named entities. The entities in ### may be either filled using copying mechanisms - see, e.g., Gu, J., Lu, Z., Li, H. and Li, V.O., 2016.

|   | Example question template |
| --- | --- |
| I | What imaging abnormalities can be seen in ###? |
| II | What is the most likely cause of ### abnormality? |
| III | What is the most appropriate follow-up imaging modality for monitoring ###? |
| IV | Is there a ### in the ###? |

Incorporating copying mechanism in sequence-to-sequence learning. arXiv preprint arXiv:1603.06393. - or use pointer generation networks - see, e.g., See, A., Liu, P.J. and Manning, C.D., 2017. Get to the point: Summarization with pointer-generator networks. arXiv preprint arXiv:1704.04368.

Generally, instead of posing the cues as questions, the cues may be posed as notes. This is shown in TAB. 4 for the similar examples as in TAB. 3.

TAB. 4: example cues templates, here for an implementation of posing the cues as questions as in TAB. 1 example II.

|   | Example note template |
| --- | --- |
| I | The imaging abnormality ### can be seen. |
| II | The most likely root cause for ### is XYZ. |
| III | XYZ is the most likely follow-up imaging modality for monitoring ###. |

(continued)

|  | Example note template |
|---|---|
| IV | There is a ### in the ###? |

FIG. 6 illustrates a scenario in which the preprocessing algorithm 131 obtains the medical imaging datasets 113, 114 as inputs. As explained in connection with FIG. 1: it would be possible, to use a medical analytics algorithm 122 to preprocess the images and then provide the output of the medical analytics algorithm 122 as a further input to the processing algorithm 131 (or provide the output of the medical analytics algorithm 122 instead of the medical imaging datasets 113, 114 is input).

Thus, the processing algorithm 131 can optionally take in automated results generated using medical analytics algorithm. For example, given a chest X-ray image, the medical analytics algorithm can detect and characterize radiographic findings within the image. If there are any new emergent suspicious radiographic findings that were not seen in the previous timepoint image or report, this can be flagged as a diagnostic cue to the reporting radiologist.

Once the latent features 321 have been determined, the latent features can be fused in a fusing layer 331. Then, a decoder branch 341 can be used to determine the one or diagnostic cues. Thus, the latent features of multiple encoder branches can be merged to obtain merge latent features and then, a decoder branch can be used for reconstructing the one or more diagnostic cues based on the merged latent features.

As will be appreciated, where the processing algorithm 131 considers one or prior medical reports and outputs the one or diagnostic cues, the processing algorithm can be implemented by a sequence-to-sequence network, combined with an image-to-sequence network.

As a general rule, it would be possible that a fixed number of diagnostic cues is output by the processing algorithm. A random variable provided as a control input to the processing algorithm can introduce variability between different diagnostic cues. An index can toggle between different diagnostic cues. Generally, user feedback obtained in response to opposing a first diagnostic cue to the user can be used as an input for determining a subsequent, second diagnostic cue (cf. FIG. 1: feedback 190).

The processing algorithm 131 thus can be described as follows:

Given a tokenized prior medical report $X_{T-1} = (x_1 \cdots x_n)$ (cf. TAB. 2), a prior medical imaging dataset including, e.g., an image $I_{T-1}$ and a current medical imaging dataset including, e.g., a current image $I_T$, the processing algorithm 131 generates cues $Y_T = (y_1 \cdots y_m)$ which ae defined as the best cues $\hat{Y}_T$ that maximizes the condition likelihood given $X_{T-1}$, $I_{T-1}$, $I_T$ :

$$\hat{Y}_T = \arg max_Y \, P(Y \,|X_{T-1}, I_{T-1}, I_T \,) \;=\; \arg max_Y \sum_{t=1}^{m} P(y_t \,|X_{T-1}, I_{T-1}, I_T, y_{<t} \,)$$

P can be modeled using a hybrid architecture with an RNN-based encoder for modeling $X_{T-1}$, CNN-based encoder for $I_{T-1}$ and $I_T$ and an RNN-based decoder for $\hat{Y}_T$.

The training phase 2005 (cf. FIG. 3) of such processing algorithm can be implemented as follows:

Multiple training datasets can be obtained. Each training dataset can include respective training medical imaging datasets and optionally respective training medical reports.

Training datasets can be obtained for different imaging modalities and/or different imaged anatomical regions. Training datasets can be obtained for different imaging equipment. The training datasets can be harmonized to minimize inter-site variations. Quality control can be implemented based with expert medical professionals. The training datasets could be anonymized. The training medical imaging datasets can be matched to the training medical reports. This corresponds to various inputs to the processing algorithm 131, as discussed in connection with FIG.6.

Then, experts can manually determine diagnostic cues for the multiple training datasets. This corresponds to ground-truth label generation. For instance, actionable questions can be selected by multiple experts from relevant question templates to generate a corpus of expert verified report - question pairs.

By this, pairs of input and output data as ground truth for the processing algorithm 131 are obtained, for the training datasets.

Then, the training can be implemented using conventional training techniques, e.g., gradient descent and backpropagation. Loss functions such as cross-entropy loss, KL-divergence, question reconstruction loss and regularization loss functions can be used.

The trained processing algorithm could then be validated, e.g., based on unseen-held-out training datasets. Various validation metrics are conceivable, e.g., BLEU, METEOR, ROGUE etc.

In some examples, LLL can be employed (cf. FIG. 3, feedback loop; FIG. 5: box 4050). This means that posing the one or more diagnostic cues to the user during the inference phase 2010 can be operated in a closed loop with the training phase 2005 (cf. FIG. 3). The ground-truth labels could be either explicit radiologist feedback such a satisfaction with generated questions, 'like' button or scoring like a satisfaction scale. Alternatively, or additionally, indirect performance and quality metrics would be concise such as reduced hedging, detailed reports, improved quality of reporting in anonymous peer review etc. Such a feedback can be used to improve the question generation using methods for weak supervision, reinforcement learning etc. thus, it would be possible to obtain user feedback associated with the one or more diagnostic cues. The user feedback can indicate a quality or relevance of each one of the one or diagnostic cues. Then, at least a part of the processing algorithm 131 can be retrained based on a user feedback.

Next, further details with respect to taking into account one or more prior medical reports will be discussed in connection with the following FIGs.

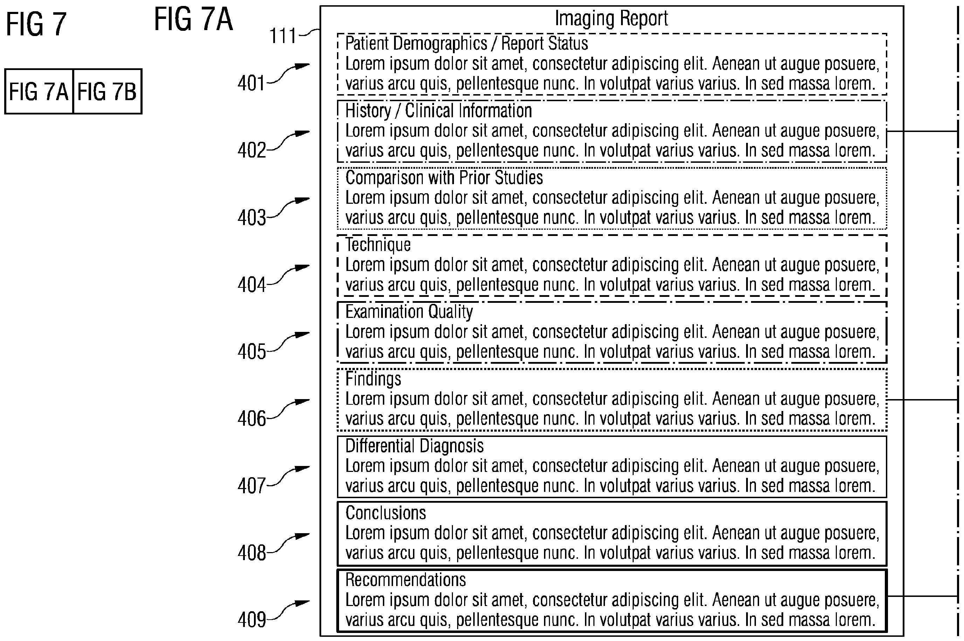

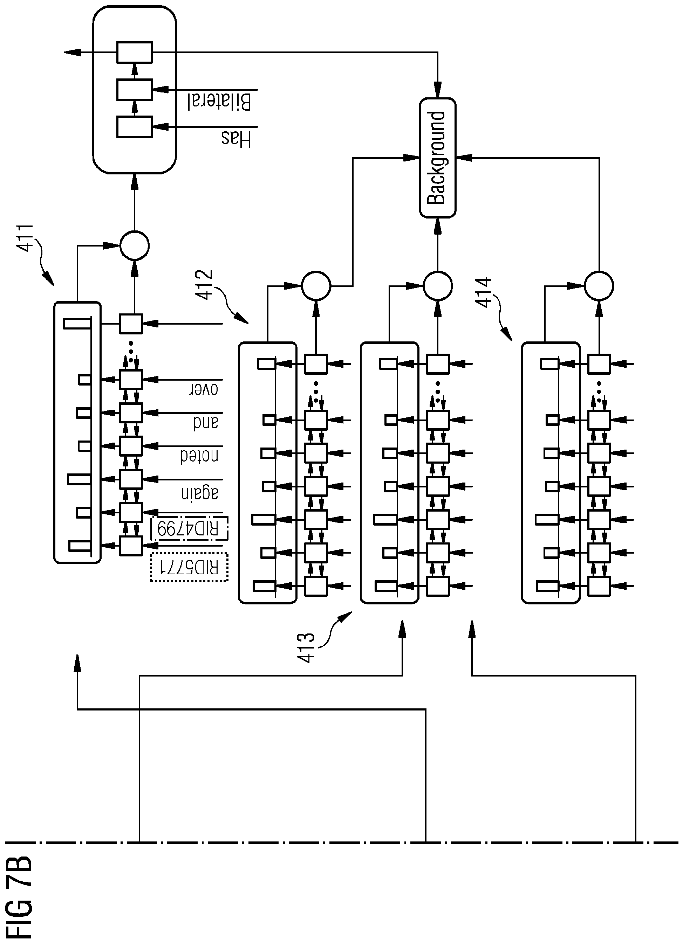

FIG. 7 illustrates aspects with respect to the prior medical report 111. The prior medical report includes multiple sections such as a patient demographics section 401, a clinical history section 402 of the patient, a section 403 pertaining to a comparison of the medical examination subject to the prior medical report 111 with one or more further prior examinations, a technique section 404, a section 405 outlining the quality of the medical examination underlying the prior medical report 111, a findings section 406, a diagnosis section 407, a conclusion section 408, and a recommendation section 409.

Other medical reports may include other sections, or fewer sections, or additional sections. FIG. 7 is only an example.

FIG. 7 also illustrates aspects with respect to processing the multiple sections 401-409 using the processing algorithm 131.

As a general rule: Multiple encoder branches 411-414 can be used to process different sections of the prior medical report 111, e.g., a machine-readable representation thereof. The different encoder branches may be trained separately or jointly.

The different sections can thus, generally, provided to different input channels of the processing algorithm 131.

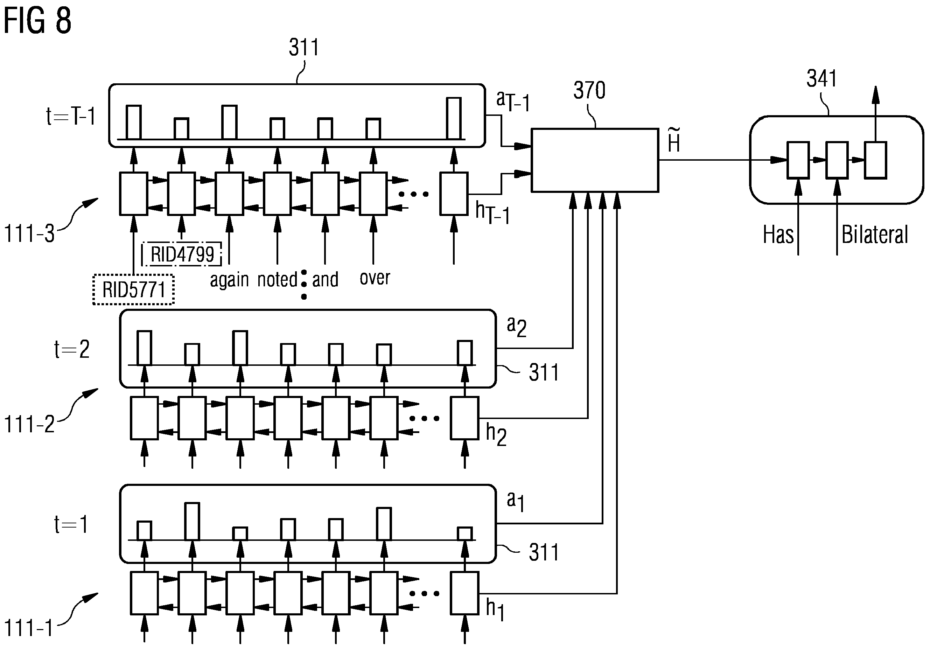

Above, in connection with FIG. 6 and FIG. 7, scenarios have been explained in which a single prior medical report 111 (or, respectively, the machine-readable representation 112 thereof) is used as an input to the processing algorithm 131. As a general rule (and as explained at high generality in connection with FIG. 4), it would be possible to obtain multiple prior medical reports of multiple prior examinations of the patient. It would be possible that the processing algorithm 131 processes all these multiple prior medical reports. A corresponding example is illustrated in FIG. 8.

FIG. 8 illustrates aspects with respect to the processing algorithm 131 processing multiple prior medical reports 111-1, 111-2, 111-3. Here, three prior reports 111-1 - 111-3 are obtained, for multiple prior time points. Each prior medical report is processed in a respective instance of the encoder branch 311. Then, a multiple-instance pooling layer is 370 used to merge the respective latent features encoding each one of the prior reports 111-1 - 111-3 using the encoder branch 311.

The multiple-instance pooling facilitates fusion of latent features associated with an arbitrary number of prior medical reports.

For instance, in typical routine chest X-ray monitoring scenarios, a series of medical reports and images are collected over time. The knowledge from all the prior scans (t<T) can be used as input to the processing algorithm 131 in timepoint T.

This can be implemented using multiple-instance learning. The multiple-instance pooling layer 370 can be realized using differentiable pooling functions such as Noisy-OR, log-sum exponentiation, max-pooling, softmax pooling, noisy-AND pooling, generalized mean-pooling, etc.

The medical-instance pooling layer can include attention weighting for the multiple medical reports 111-1 -111-3.

Hence, it is possible to combine an attention mechanism with multiple instance pooling. The hidden state of the report encoders for predict at time $T$ can be represented as $H = (h_1 \cdots h_{T-1})$ and the corresponding attention matrix as $A = (a_1 \cdots a_{T-1})$. The hidden vectors fed into the RNN-decoder 341 for question generation are defined as $\tilde{H} = max_T A \odot H$ where $\odot$ is element-wise multiplication and max operator. Alternative mechanisms such as time-base attention, interaction base attention etc. can also be used, see, e.g., Ma, F., Chitta, R., Zhou, J., You, Q., Sun, T. and Gao, J., 2017, August. Dipole: Diagnosis prediction in healthcare via attention-based bidirectional recurrent neural networks. In Proceedings of the 23rd ACM SIGKDD international conference on knowledge discovery and data mining (pp. 1903-1911).

Summarizing, various techniques have been disclosed which facilitate drawing up a medical report by a medical practitioner. This is based on one more diagnostic cues that are posed to the user during a workflow for drawing up the medical report.

Optionally, such one or more diagnostic cues may also be posed to a patient so that the patient can use the one or more diagnostic cues to analyze what the right questions is to ask their caregiver.

Optionally, the same technique can be applied to other tasks beyond medical report preparation, e.g., tumor-boards, therapy planning, patient monitoring, screening etc.

Summarizing, at least the following EXAMPLES have been disclosed:

EXAMPLE 1. A computer-implemented method, comprising:

- obtaining (4020) medical imaging dataset (113) of a current examination of a patient,
- based on the medical imaging dataset (113), determining one or more di-agnostic cues (119) using a processing algorithm, the one or more diagnostic cues (119) being associated with patient-specific diagnostic findings, and
- as part of a workflow for drawing up a current medical report (145) for the current examination, controlling (4035) a user interface (90) to pose the one or more diagnostic cues (119) to a user.

EXAMPLE 2. The computer-implemented method of EXAMPLE 1,
wherein the one or more diagnostic cues (119) are posed as questions,
wherein the method further comprises:

- obtaining, from the user interface (90), a user feedback (119) to at least one of the one or more diagnostic cues, and
- editing the current medical report (145) based on the user feedback.

EXAMPLE 3. The computer-implemented method of EXAMPLE 2,
wherein said editing of the current medical report based on the user feedback comprises generating the medical report or refining the current medical report.

EXAMPLE 4. The computer-implemented method of any one of the preceding EXAMPLEs,
wherein the user interface (90) is controlled to pose the one or more diagnostic cues (119) to the user prior to a generation step of the workflow.

EXAMPLE 5. The computer-implemented method of any one of EXAMPLEs 1 to 3,
wherein the user interface is controlled to pose the one or more diagnostic cues (119) to the user after a generation step of the workflow as part of which the current medical report is generated and during a validation step of the workflow providing a validation toolset to the user for refining the current medical report (145), wherein the one or more diagnostic cues are optionally determined further based on the current medical report.

EXAMPLE 6. The computer-implemented method of any one of the preceding EXAMPLEs, further comprising:

- obtaining (4005) at least one prior medical report (111) of at least one prior examination of the patient, and
- using a natural language processing algorithm (121), analyzing the at least one prior medical report (111) to obtain a machine-readable representation (112) of the at least one prior medical report (111),

wherein the one or more diagnostic cues (119) are determined based on the machine-readable representation (112) of the at least one prior medical report (111-3).

EXAMPLE 7. The computer-implemented method of EXAMPLE 6,

wherein the machine-readable representation (112-2) of the at least one prior medical report (111) classifies multiple sections (401 - 409) of the at least one prior medical report (111),
wherein different ones of the multiple sections (401 - 409) are provided to different input channels of the processing algorithm (131), the different input channels being optionally associated with different encoder branches (411 - 414) of the processing algorithm (131).

EXAMPLE 8. The computer-implemented method of any one of the preceding EXAMPLEs, further comprising:

- analyzing (4025) the medical imaging dataset (113) of the current examination using a medical analytics algorithm (122) to determine result data for the medical imaging dataset (113),

wherein the one or more diagnostic cues (119) are determined based on the result data.

EXAMPLE 9. The computer-implemented method of any one of the preceding EXAMPLEs, further comprising:

- obtaining (4015) at least one prior medical imaging dataset (114) of at least one prior examination,

wherein the one or more diagnostic cues (119) are determined further based on the at least one prior medical imaging dataset (114) of the at least one prior examination.

EXAMPLE 10. The computer-implemented method of any one of the preceding EXAMPLEs, further comprising:

- obtaining at least one prior medical report (111) of at least one prior examination of the patient,

wherein the processing algorithm is machine-learned and comprises multiple encoder branches (301, 302, 311) configured to determine respective latent features, different encoder branches (301, 302, 311) being associated with the at least one prior medical report and the medical imaging dataset (113) of the current examination.

EXAMPLE 11. The computer-implemented method of EXAMPLE 10,
wherein a first encoder branch (311) of the multiple encoder branches associated with the at least one prior medical report (111) comprises a re-current neural network such as a bi-directional long-short-term memory encoder branch.

EXAMPLE 12. The computer-implemented method of EXAMPLE 10 or 11,
wherein a second encoder branch (301, 302) of the multiple encoder branches associated with the medical imaging dataset (113) of the current examination comprises a convolutional neural network.

EXAMPLE 13. The computer-implemented method of any one of EXAMPLEs 10 to 12,
wherein at least one encoder branch (311-3) of the multiple encoder branches associated with the at least one prior medical report comprises an attention layer for determining shortcuts between the respective latent features (321) and a respective input to the processing algorithm (131) .

EXAMPLE 14. The computer-implemented method of any one of EXAMPLEs 10 to 13,
wherein the processing algorithm (131) merges (331) the latent features (321) of the multiple encoder branches (301, 302, 311), to obtain merged latent features,
wherein the processing algorithm (131-4) comprises a decoder branch (341) for reconstructing the one or more diagnostic cues based on the merged latent features.

EXAMPLE 15. The computer-implemented method of any one of EXAMPLEs 10 to 14, further comprising:

- obtaining multiple prior medical reports (111) of multiple prior examinations of the patient and

wherein the processing algorithm (131) comprises a multiple-instance pooling layer (370) to merge latent features obtained from a respective encoder branch (311) of the multiple encoder branches used to encode each one of the multiple prior medical reports (111).

EXAMPLE 16. The computer-implemented method of EXAMPLE 15,
wherein the multiple-instance pooling layer (370) comprises attention weighting for the multiple prior medical reports (111).

EXAMPLE 17. The computer-implemented method of any one of EXAMPLEs 10 to 15, further comprising:

- obtaining (4050) a user feedback associated with the one or more diagnostic cues, and
- re-training at least a part of the processing algorithm (131) based on the user feedback.

EXAMPLE 18. The computer-implemented method of any one of the preceding EXAMPLEs,

wherein multiple diagnostic cues (1190) are sequentially determined,
wherein the method further comprises:

- obtaining a user feedback (190) associated with the multiple cues,

wherein said controlling of the user interface (90), said determining of the multiple diagnostic cues, and said obtaining of the user feedback (190) is implemented in an entangled manner, so that a subsequent diagnostic cue (119) of the multiple diagnostic cues (119) depends on the user feedback (190) associated with a preceding cue (119).

EXAMPLE 19. The computer-implemented method of any one of the preceding EXAMPLEs, wherein the processing algorithm (131-) determines the one or more diagnostic cues (119) in accordance with an institution-specific reporting guideline for drawing up medical reports.

EXAMPLE 20. A device (91) comprising a processer (92) configured to perform the method of any one of the preceding EXAMPLES.

EXAMPLE 21. A computer program or a computer program code that is executable by a processor (92), wherein execution of the program code causes the processor to:

- obtain (4020) medical imaging dataset (113) of a current examination of a patient,
- based on the medical imaging dataset (113), determine one or more diagnostic cues (119) using a processing algorithm, the one or more diagnostic cues (119) being associated with patient-specific diagnostic findings, and
- as part of a workflow for drawing up a current medical report (145) for the current examination, control (4035) a user interface (90) to pose the one or more diagnostic cues (119) to a user.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

**Claims**

1. A computer-implemented method, comprising:

   - obtaining (4020) medical imaging dataset (113) of a current examination of a patient,
   - based on the medical imaging dataset (113), determining one or more diagnostic cues (119) using a processing algorithm, the one or more diagnostic cues (119) being associated with patient-specific diagnostic findings, and
   - as part of a workflow for drawing up a current medical report (145) for the current examination, controlling (4035) a user interface (90) to pose the one or more diagnostic cues (119) to a user.

2. The computer-implemented method of claim 1,
   wherein the one or more diagnostic cues (119) are posed as questions,
   wherein the method further comprises:

   - obtaining, from the user interface (90), a user feedback (119) to at least one of the one or more diagnostic cues, and
   - editing the current medical report (145) based on the user feedback.

3. The computer-implemented method of claim 2,
   wherein said editing of the current medical report based on the user feedback comprises generating the medical report or refining the current medical report.

4. The computer-implemented method of any one of the preceding claims,
   wherein the user interface is controlled to pose the one or more diagnostic cues (119) to the user after a generation step of the workflow as part of which the current medical report is generated and during a validation step of the workflow providing a validation toolset to the user for refining the current medical report (145), wherein the one or more diagnostic cues are optionally determined further based on the current medical report.

5. The computer-implemented method of any one of the preceding claims, further comprising:

   - obtaining (4005) at least one prior medical report (111) of at least one prior examination of the patient, and
   - using a natural language processing algorithm (121), analyzing the at least one prior medical report (111) to obtain a machine-readable representation (112) of the at least one prior medical report (111),

wherein the one or more diagnostic cues (119) are determined based on the machine-readable representation (112) of the at least one prior medical report (111-3).

6. The computer-implemented method of claim 5,

   wherein the machine-readable representation (112-2) of the at least one prior medical report (111) classifies multiple sections (401 - 409) of the at least one prior medical report (111),
   wherein different ones of the multiple sections (401 - 409) are provided to different input channels of the processing algorithm (131), the different input channels being optionally associated with different encoder branches (411 - 414) of the processing algorithm (131).

7. The computer-implemented method of any one of the preceding claims, further comprising:

   - obtaining (4015) at least one prior medical imaging dataset (114) of at least one prior examination,
   wherein the one or more diagnostic cues (119) are determined further based on the at least one prior medical imaging dataset (114) of the at least one prior examination.

8. The computer-implemented method of any one of the preceding claims, further comprising:

   - obtaining at least one prior medical report (111) of at least one prior examination of the patient,
   wherein the processing algorithm is machine-learned and comprises multiple encoder branches (301, 302, 311) configured to determine respective latent features, different encoder branches (301, 302, 311) being associated with the at least one prior medical report and the medical imaging dataset (113) of the current examination.

9. The computer-implemented method of claim 8,
   wherein at least one encoder branch (311-3) of the multiple encoder branches associated with the at least one prior medical report comprises an attention layer for determining shortcuts between the respective latent features (321) and a respective input to the processing algorithm (131) .

10. The computer-implemented method of claim 8 or 9,

    wherein the processing algorithm (131) merges (331) the latent features (321) of the multiple encoder branches (301, 302, 311), to obtain merged latent features,
    wherein the processing algorithm (131-4) comprises a decoder branch (341) for reconstructing the one or more diagnostic cues based on the merged latent features.

11. The computer-implemented method of any one of claims 8 to 10, further comprising:

    - obtaining multiple prior medical reports (111) of multiple prior examinations of the patient and
    wherein the processing algorithm (131) comprises a multiple-instance pooling layer (370) to merge latent features obtained from a respective encoder branch (311) of the multiple encoder branches used to encode each one of the multiple prior medical reports (111).

12. The computer-implemented method of claim 11,
    wherein the multiple-instance pooling layer (370) comprises attention weighting for the multiple prior medical reports (111).

13. The computer-implemented method of any one of the preceding claims,

    wherein multiple diagnostic cues (1190) are sequentially determined,
    wherein the method further comprises:

    - obtaining a user feedback (190) associated with the multiple cues,

    wherein said controlling of the user interface (90), said determining of the multiple diagnostic cues, and said obtaining of the user feedback (190) is implemented in an entangled manner, so that a subsequent diagnostic cue (119) of the multiple diagnostic cues (119) depends on the user feedback (190) associated with a preceding cue (119).

**14.** A device (91) comprising a processer (92) configured to:

- obtain (4020) medical imaging dataset (113) of a current examination of a patient,
- based on the medical imaging dataset (113), determine one or more diagnostic cues (119) using a processing algorithm, the one or more diagnostic cues (119) being associated with patient-specific diagnostic findings, and
- as part of a workflow for drawing up a current medical report (145) for the current examination, control (4035) a user interface (90) to pose the one or more diagnostic cues (119) to a user.

**15.** A computer program or a computer program product comprising program code that is executable by a processor (92), wherein execution of the program code causes the processor to:

- obtain (4020) medical imaging dataset (113) of a current examination of a patient,
- based on the medical imaging dataset (113), determine one or more diagnostic cues (119) using a processing algorithm, the one or more diagnostic cues (119) being associated with patient-specific diagnostic findings, and
- as part of a workflow for drawing up a current medical report (145) for the current examination, control (4035) a user interface (90) to pose the one or more diagnostic cues (119) to a user.

**Amended claims in accordance with Rule 137(2) EPC.**

**1.** A computer-implemented method, comprising:

- obtaining (4020) medical imaging dataset (113) of a current examination of a patient,
- based on the medical imaging dataset (113), determining one or more diagnostic cues (119) using a processing algorithm, the one or more diagnostic cues (119) being associated with patient-specific diagnostic findings, and
- as part of a workflow for drawing up a current medical report (145) for the current examination, controlling (4035) a user interface (90) to pose the one or more diagnostic cues (119) to a user

wherein the user interface (90) is controlled to pose the one or more diagnostic cues (119) to the user after a generation step of the workflow as part of which the current medical report is generated and during a validation step of the workflow providing a validation toolset to the user for refining the current medical report (145).

**2.** The computer-implemented method of claim 1,
wherein the one or more diagnostic cues (119) are posed as questions,
wherein the method further comprises:

- obtaining, from the user interface (90), a user feedback (119) to at least one of the one or more diagnostic cues, and
- editing the current medical report (145) based on the user feedback.

**3.** The computer-implemented method of claim 2,
wherein said editing of the current medical report based on the user feedback comprises generating the medical report or refining the current medical report.

**4.** The computer-implemented method of any one of the preceding claims,
wherein the one or more diagnostic cues are determined further based on the current medical report.

**5.** The computer-implemented method of any one of the preceding claims, further comprising:

- obtaining (4005) at least one prior medical report (111) of at least one prior examination of the patient, and
- using a natural language processing algorithm (121), analyzing the at least one prior medical report (111) to obtain a machine-readable representation (112) of the at least one prior medical report (111),
wherein the one or more diagnostic cues (119) are determined based on the machine-readable representation (112) of the at least one prior medical report (111-3).

**6.** The computer-implemented method of claim 5,

wherein the machine-readable representation (112-2) of the at least one prior medical report (111) classifies multiple sections (401 - 409) of the at least one prior medical report (111),
wherein different ones of the multiple sections (401 - 409) are provided to different input channels of the processing algorithm (131), the different input channels being optionally associated with different encoder branches (411 - 414) of the processing algorithm (131).

7. The computer-implemented method of any one of the preceding claims, further comprising:

- obtaining (4015) at least one prior medical imaging dataset (114) of at least one prior examination,
wherein the one or more diagnostic cues (119) are determined further based on the at least one prior medical imaging dataset (114) of the at least one prior examination.

8. The computer-implemented method of any one of the preceding claims, further comprising:

- obtaining at least one prior medical report (111) of at least one prior examination of the patient,
wherein the processing algorithm is machine-learned and comprises multiple encoder branches (301, 302, 311) configured to determine respective latent features, different encoder branches (301, 302, 311) being associated with the at least one prior medical report and the medical imaging dataset (113) of the current examination.

9. The computer-implemented method of claim 8,
wherein at least one encoder branch (311-3) of the multiple encoder branches associated with the at least one prior medical report comprises an attention layer for determining shortcuts between the respective latent features (321) and a respective input to the processing algorithm (131).

10. The computer-implemented method of claim 8 or 9,

wherein the processing algorithm (131) merges (331) the latent features (321) of the multiple encoder branches (301, 302, 311), to obtain merged latent features,
wherein the processing algorithm (131-4) comprises a decoder branch (341) for reconstructing the one or more diagnostic cues based on the merged latent features.

11. The computer-implemented method of any one of claims 8 to 10, further comprising:

- obtaining multiple prior medical reports (111) of multiple prior examinations of the patient and
wherein the processing algorithm (131) comprises a multiple-instance pooling layer (370) to merge latent features obtained from a respective encoder branch (311) of the multiple encoder branches used to encode each one of the multiple prior medical reports (111).

12. The computer-implemented method of claim 11,
wherein the multiple-instance pooling layer (370) comprises attention weighting for the multiple prior medical reports (111).

13. The computer-implemented method of any one of the preceding claims,

wherein multiple diagnostic cues (1190) are sequentially determined,
wherein the method further comprises:

- obtaining a user feedback (190) associated with the multiple cues,

wherein said controlling of the user interface (90), said determining of the multiple diagnostic cues, and said obtaining of the user feedback (190) is implemented in an entangled manner, so that a subsequent diagnostic cue (119) of the multiple diagnostic cues (119) depends on the user feedback (190) associated with a preceding cue (119).

14. A device (91) comprising a processer (92) configured to:

- obtain (4020) medical imaging dataset (113) of a current examination of a patient,
- based on the medical imaging dataset (113), determine one or more diagnostic cues (119) using a processing algorithm, the one or more diagnostic cues (119) being associated with patient-specific diagnostic findings, and

- as part of a workflow for drawing up a current medical report (145) for the current examination, control (4035) a user interface (90) to pose the one or more diagnostic cues (119) to a user

wherein the user interface (90) is controlled to pose the one or more diagnostic cues (119) to the user after a generation step of the workflow as part of which the current medical report is generated and during a validation step of the workflow providing a validation toolset to the user for refining the current medical report (145).

15. A computer program or a computer program product comprising program code that is executable by a processor (92), wherein execution of the program code causes the processor to:

- obtain (4020) medical imaging dataset (113) of a current examination of a patient,
- based on the medical imaging dataset (113), determine one or more diagnostic cues (119) using a processing algorithm, the one or more diagnostic cues (119) being associated with patient-specific diagnostic findings, and
- as part of a workflow for drawing up a current medical report (145) for the current examination, control (4035) a user interface (90) to pose the one or more diagnostic cues (119) to a user

wherein the user interface (90) is controlled to pose the one or more diagnostic cues (119) to the user after a generation step of the workflow as part of which the current medical report is generated and during a validation step of the workflow providing a validation toolset to the user for refining the current medical report (145).

FIG 1

FIG 2

FIG 3

FIG 4

FIG 5

2010

4005

4010

4015

4020

4025

4030

4035

4040

4045

2005

4050

FIG 6

**FIG 7**

| FIG 7A | FIG 7B |
|--------|--------|

**FIG 7A**

111

**Imaging Report**

401 — **Patient Demographics / Report Status**
Lorem ipsum dolor sit amet, consectetur adipiscing elit. Aenean ut augue posuere, varius arcu quis, pellentesque nunc. In volutpat varius varius. In sed massa lorem.

402 — **History / Clinical Information**
Lorem ipsum dolor sit amet, consectetur adipiscing elit. Aenean ut augue posuere, varius arcu quis, pellentesque nunc. In volutpat varius varius. In sed massa lorem.

403 — **Comparison with Prior Studies**
Lorem ipsum dolor sit amet, consectetur adipiscing elit. Aenean ut augue posuere, varius arcu quis, pellentesque nunc. In volutpat varius varius. In sed massa lorem.

404 — **Technique**
Lorem ipsum dolor sit amet, consectetur adipiscing elit. Aenean ut augue posuere, varius arcu quis, pellentesque nunc. In volutpat varius varius. In sed massa lorem.

405 — **Examination Quality**
Lorem ipsum dolor sit amet, consectetur adipiscing elit. Aenean ut augue posuere, varius arcu quis, pellentesque nunc. In volutpat varius varius. In sed massa lorem.

406 — **Findings**
Lorem ipsum dolor sit amet, consectetur adipiscing elit. Aenean ut augue posuere, varius arcu quis, pellentesque nunc. In volutpat varius varius. In sed massa lorem.

407 — **Differential Diagnosis**
Lorem ipsum dolor sit amet, consectetur adipiscing elit. Aenean ut augue posuere, varius arcu quis, pellentesque nunc. In volutpat varius varius. In sed massa lorem.

408 — **Conclusions**
Lorem ipsum dolor sit amet, consectetur adipiscing elit. Aenean ut augue posuere, varius arcu quis, pellentesque nunc. In volutpat varius varius. In sed massa lorem.

409 — **Recommendations**
Lorem ipsum dolor sit amet, consectetur adipiscing elit. Aenean ut augue posuere, varius arcu quis, pellentesque nunc. In volutpat varius varius. In sed massa lorem.

EP 4 141 878 A1

FIG 7B

Bilateral

Has

Background

over
and
noted
again
RID4799
RID5771

411

412

413

414

# FIG 8

311

t=T-1    $a_{T-1}$    370    341    $\tilde{H}$

111-3    $h_{T-1}$

RID4799

RID5771

again noted : and    over

Has    Bilateral

t=2    $a_2$    311

111-2    $h_2$

t=1    $a_1$    311

111-1    $h_1$

EP 4 141 878 A1

EUROPEAN SEARCH REPORT

Application Number

EP 21 19 3589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/159737 A1 (BUCKLER ANDREW J [US] ET AL) 30 May 2019 (2019-05-30) | 1-4,8-15 | INV. G16H15/00 |
| Y | * paragraph [0121] - paragraph [0125] *<br>* paragraph [0059] - paragraph [0060] *<br>* paragraph [0075] *<br>* paragraph [0136] *<br>* paragraph [0137] *<br>* paragraph [0195] *<br>* paragraph [0396] *<br>* paragraph [0128] *<br>* paragraph [0404] *<br>* paragraph [0405] *<br>* paragraph [0153] *<br>* paragraph [0038] - paragraph [0039] *<br>* paragraph [0040] *<br>* paragraph [0094] *<br>* paragraph [0162] * | 5-7 | |
| X | WO 2019/033098 A2 (ELUCID BIOIMAGING INC [US]) 14 February 2019 (2019-02-14)<br>* figure 4 *<br>* page 16, line 12 - page 18, line 14 * | 1,14,15 | |
| X | US 2021/209757 A1 (MIN JAMES K [US] ET AL) 8 July 2021 (2021-07-08)<br>* paragraph [0273] *<br>* paragraph [0272] *<br>* paragraph [0433] - paragraph [0435] * | 1,14,15 | TECHNICAL FIELDS SEARCHED (IPC)<br><br>G16H |
| X | WO 2019/103912 A2 (ARTERYS INC [US]; GOLDEN DANIEL IRVING [US] ET AL.) 31 May 2019 (2019-05-31)<br>* page 78, line 1 - page 79, line 13 * | 1,14,15 | |

-/--

The present search report has been drawn up for all claims

| Place of search | Date of completion of the search | Examiner |
|---|---|---|
| Munich | 15 February 2022 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 21 19 3589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/222755 A1 (ARTERYS INC [US]; TAERUM TORIN ARNI [CA]; JUGDEV TRISTAN [CA]) 6 December 2018 (2018-12-06)<br>* figure 4 *<br>* page 16, line 12 – page 18, line 14 *<br>----- | 1,14,15 | |
| Y | US 2012/221347 A1 (REINER BRUCE [US]) 30 August 2012 (2012-08-30)<br>* paragraph [0071] – paragraph [0072] *<br>* paragraph [0078] *<br>----- | 5-7 | |

**TECHNICAL FIELDS SEARCHED (IPC)**

The present search report has been drawn up for all claims

| Place of search | Date of completion of the search | Examiner |
|---|---|---|
| Munich | 15 February 2022 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 3589

This annex lists the patent family members relating to the patent documents cited in the above-mentioned European search report.
The members are as contained in the European Patent Office EDP file on
The European Patent Office is in no way liable for these particulars which are merely given for the purpose of information.

15-02-2022

| Patent document cited in search report | | Publication date | Patent family member(s) | | Publication date |
|---|---|---|---|---|---|
| US 2019159737 | A1 | 30-05-2019 | US 2019159737 | A1 | 30-05-2019 |
| | | | US 2021282719 | A1 | 16-09-2021 |
| WO 2019033098 | A2 | 14-02-2019 | EP 3665702 | A2 | 17-06-2020 |
| | | | US 2019074082 | A1 | 07-03-2019 |
| | | | WO 2019033098 | A2 | 14-02-2019 |
| US 2021209757 | A1 | 08-07-2021 | US 2021209757 | A1 | 08-07-2021 |
| | | | US 2021217165 | A1 | 15-07-2021 |
| | | | US 2021241454 | A1 | 05-08-2021 |
| | | | US 2021241455 | A1 | 05-08-2021 |
| | | | US 2021256693 | A1 | 19-08-2021 |
| | | | US 2021256694 | A1 | 19-08-2021 |
| | | | US 2021256695 | A1 | 19-08-2021 |
| | | | US 2021366111 | A1 | 25-11-2021 |
| | | | US 2021366112 | A1 | 25-11-2021 |
| | | | US 2021366113 | A1 | 25-11-2021 |
| | | | US 2021366115 | A1 | 25-11-2021 |
| | | | US 2021366116 | A1 | 25-11-2021 |
| | | | US 2022028070 | A1 | 27-01-2022 |
| | | | WO 2021141921 | A1 | 15-07-2021 |
| WO 2019103912 | A2 | 31-05-2019 | EP 3714467 | A2 | 30-09-2020 |
| | | | US 2020380675 | A1 | 03-12-2020 |
| | | | WO 2019103912 | A2 | 31-05-2019 |
| WO 2018222755 | A1 | 06-12-2018 | EP 3629898 | A1 | 08-04-2020 |
| | | | US 2020085382 | A1 | 19-03-2020 |
| | | | WO 2018222755 | A1 | 06-12-2018 |
| US 2012221347 | A1 | 30-08-2012 | NONE | | |

EPO FORM P0459

For more details about this annex : see Official Journal of the European Patent Office, No. 12/82

**EP 4 141 878 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PENNINGTON, J. ; SOCHER, R. ; MANNING, C.D.** Glove: Global vectors for word representation. *Proceedings of the 2014 conference on empirical methods in natural language processing (EMNLP),* October 2014, 1532-1543
- **LEE, J. ; YOON, W. ; KIM, S. ; KIM, D. ; KIM, S. ; SO, C.H. ; KANG, J.** BioBERT: a pre-trained biomedical language representation model for biomedical text mining. *Bioinformatics,* 2020, vol. 36 (4), 1234-1240

- **YU, Z. ; YU, J. ; CUI, Y. ; TAO, D. ; TIAN, Q.** Deep modular co-attention networks for visual question answering. *Proceedings of the IEEE/CVF Conference on Computer Vision and Pattern Recognition,* 2019, 6281-6290
- **A., LIU, P.J. ; MANNING, C.D.** *Get to the point: Summarization with pointer-generator networks,* 2017
- **MA, F. ; CHITTA, R. ; ZHOU, J. ; YOU, Q. ; SUN, T. ; GAO, J.** Dipole: Diagnosis prediction in healthcare via attention-based bidirectional recurrent neural networks. *Proceedings of the 23rd ACM SIGKDD international conference on knowledge discovery and data mining,* August 2017, 1903-1911